(19) Europäisches Patentamt
European Patent Office
Office européen des brevets

(11) **EP 2 995 247 B1**

(12) **EUROPEAN PATENT SPECIFICATION**

(45) Date of publication and mention
of the grant of the patent:
**26.12.2018 Bulletin 2018/52**

(51) Int Cl.:
*A61B 5/01* (2006.01)    *A61B 5/00* (2006.01)
*G01K 13/00* (2006.01)

(21) Application number: **15184452.9**

(22) Date of filing: **09.09.2015**

(54) **HEAT FLOW MEASURING APPARATUS AND METABOLISM MEASURING APPARATUS**

WÄRMEFLUSSMESSVORRICHTUNG UND STOFFWECHSELMESSVORRICHTUNG

APPAREIL DE MESURE DE FLUX THERMIQUE ET APPAREIL DE MESURE DU MÉTABOLISME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.09.2014 JP 2014184518**

(43) Date of publication of application:
**16.03.2016 Bulletin 2016/11**

(73) Proprietor: **Seiko Epson Corporation
Shinjuku-ku
Tokyo 163-0811 (JP)**

(72) Inventors:
• **Ikeda, Akira
Suwa-shi, Nagano 392-8502 (JP)**
• **Ito, Megumu
Suwa-shi, Nagano 392-8502 (JP)**

(74) Representative: **Hoffmann Eitle
Patent- und Rechtsanwälte PartmbB
Arabellastraße 30
81925 München (DE)**

(56) References cited:
**EP-A2- 2 458 356      WO-A1-2013/121762
JP-A- 2014 055 963**

**Description**

BACKGROUND

1. Technical Field

The present invention relates to a heat flow measuring apparatus which measures heat flow or the like.

2. Related Art

Measurement of a temperature or heat flow is important for estimating the state of a measurement target.

For example, it is possible to check health through continuous monitoring of a body temperature over a long period of time or it is possible to check biological rhythm, functions of metabolism and autonomic nerves, or the like through measuring heat flow released from the trunk of a body or extremities.

As a known technology related to the measurement of the body temperature, there is a technology in which, for example, two temperature sensors adhere to a measurement target such that the body temperature is measured, and correction is performed using a difference between values from the measurement positions such that accurate measurement is realized (for example, see JP-A-2014-55963), or a technology in which strong adhesion of the temperature sensor to skin enables accuracy of measurement to be enhanced (for example, see JP-T-2007-530154).

In addition, as a known technology related to measurement of heat flow, there is a technology in which heat released from skin is guided to a heat flux sensor through a heat pipe (for example, see JP-T-2004-532065), or a technology in which an electric heater is provided around a probe that measures a skin's surface temperature and heat distribution from the skin's surface is compensated such that accuracy of measurement is enhanced (for example, see JP-A-2002-202205) .

A representative example of a heat source in a living body such as a human body includes an arterial blood vessel; however, to be more exact, positions of the arterial blood vessels are different for each individual. Accordingly, measured temperatures can be different based on positional relationships between a heat source and a temperature sensor.

As a method of compensating for a measurement difference based on the positional relationship, for example, it has been concerned to mount more temperature sensors and employ an average value obtained therefrom. However, the method is not preferable in terms of manufacturing costs or an increased size of a measurement apparatus. Particularly, in recent years, there is high demand for performing monitoring over a long period of time in a state in which the measurement apparatus is worn such that the increased size of the measurement apparatus is not preferable in terms of wearability or usability.

WO 2013/121762 A1 relates to a clinical thermometer that comprises a plurality of thermal resistors, each of which has a first temperature sensor disposed on a side that comes in contact with the surface of the body to be examined, and a second temperature sensor disposed on the side opposing the side that comes in contact with the body surface, and the clinical thermometer measures the core body temperature of the body to be examined when adhered to the surface of the body to be examined. The clinical thermometer is provided with: a first thermal resistor having a prescribed thermal resistance between the first and second temperature sensors; second and third thermal resistors having thermal resistances between the first and second temperature sensors lower than that of the first thermal resistor; a homogenization member that covers the faces of the first to third thermal resistors at the side opposing the side that comes in contact with the body surface; a selecting means for selecting either a first pair comprising the first and second thermal resistors or a second pair comprising the first and third thermal resistors, on the basis of the relationships between the temperatures of the first to third thermal resistors measured with the first temperature sensors; and a calculation means that calculates the core body temperature on the basis of the results of measurements by the temperature sensors belonging to the pair selected by the selecting means.

SUMMARY

An advantage of some aspects of the invention is to provide a technology in which it is possible to compensate for a difference in a measured temperature due to a positional relationship between a heat source and a temperature sensor.

A first aspect of the invention is directed to a heat flow measuring apparatus including: an annular first heat diffusing member; a first temperature sensor that is thermally connected to the first heat diffusing member; an annular second heat diffusing member; a second temperature sensor that is thermally connected to the second heat diffusing member; and a heat transfer layer that is disposed between the first heat diffusing member and the second heat diffusing member.

**EP 2 995 247 B1**

According to the first aspect, in a configuration in which heat flow is measured using the transfer of heat from the first heat diffusing member to the second heat diffusing member through the heat transfer layer or reverse transfer, the heat diffusing effects cause the temperature of the first heat diffusing member or the second heat diffusing member to be uniform even when positional irregularity occurs in transferring heat to the first heat diffusing member or to the second heat diffusing member. Therefore, it is possible to perform temperature measurement and heat flow measurement without the influence of the positional relationship between a heat source and the first temperature sensor or a positional relationship between the heat source and the second temperature sensor.

According to the invention, the thermal conductivity of the first heat diffusing member is higher than the thermal conductivity of the heat transfer layer, and the thermal conductivity of the second heat diffusing member is higher than the thermal conductivity of the heat transfer layer.

According to the invention, it is possible to ensure a sufficient period of time to maintain uniform temperature due to the heat diffusing effect of the first heat diffusing member until transfer is performed from the first heat diffusing member to the second heat diffusing member. Thus, irregular transferring is suppressed from the first heat diffusing member to the second heat diffusing member and further, the measurement is unlikely to be influenced by the relative positional relationship between the heat source and the temperature sensor.

As a second aspect of the invention, it is preferable that the heat flow measuring apparatus according to the first or second aspect is configured such that the thermal conductivity of the first heat diffusing member and the thermal conductivity of the second heat diffusing member are 100 (W/(m·K)) or more and the thermal conductivity of the heat transfer layer is 0.3 (W/(m·K)) to 100 (W/(m-K)).

When the heat flow measuring apparatus is configured in a structure in which an outer circumference of extremities of the measurement target (for example, a finger) like a finger ring, the heat flow measuring apparatus according to any one of the first and second aspects in which the first heat diffusing member has a heat receiving area which is in contact with a measurement target and is 204 (mm$^2$) to 690 (mm$^2$) can be configured.

According to this third aspect, since an annular shape which is properly fitted as a knuckle ring depending on various sizes of fingers is formed such that the apparatus is good in terms of wearability and usability. Thus, the ring is considered appropriate for a method in which the monitoring over a long period of time is assumed.

A fourth aspect of the invention is directed to the heat flow measuring apparatus according to any one of the first to fourth aspects, in which the maximum temperature gradient of the first heat diffusing member is larger than the maximum temperature gradient of the heat transfer layer and the maximum temperature gradient of the second heat diffusing member is larger than the maximum temperature gradient of the heat transfer layer.

According to the fourth aspect, the measurement is further unlikely to be influenced by the relative positional relationship between the heat source and the temperature sensor.

A fifth aspect of the invention is directed to the heat flow measuring apparatus according to any one of the first to sixth aspects, in which the heat flow measuring apparatus further includes: a third heat diffusing member, and a third temperature sensor that is thermally connected to the third heat diffusing member, and the third heat diffusing member and the third temperature sensor are disposed in the heat transfer layer.

According to the fifth aspect, since all of the heat diffusing effects can be enhanced, an influence of the relative positions of the heat source and the temperature sensor on measurement accuracy can be still further reduced.

A sixth aspect of the invention is directed to the heat flow measuring apparatus according to any one of the first to fifth aspects, in which a protective section that has an opening may be provided to cover the outer circumference of the second diffusing member.

According to the sixth aspect, heat releasability from the second heat diffusing member is secured and thus, it is possible to form a structure in which another object is unlikely to come into contact with the second heat diffusing member.

A seventh aspect of the invention is directed to the heat flow measuring apparatus according to the sixth aspect, in which thermal conductivity of the protective section is lower than the thermal conductivity of the heat transfer layer.

According to the seventh aspect, even in a case where an object other than the measurement target comes into contact with the protective section, there can be a low amount of influence on the measurement.

An eighth aspect of the invention is directed to a metabolism measuring apparatus including: the heat flow measuring apparatus according to any one of the first to ninth aspects; and a computing apparatus that estimates a metabolic rate based on the measurement results by the heat flow measuring apparatus.

According to the eighth aspect, it is possible to estimate metabolic rate based on highly accurate measurement results obtained by the heat flow measuring apparatus according to any one of the first to seventh aspects.

BRIEF DESCRIPTION OF THE DRAWINGS

The invention will be described with reference to the accompanying drawings, wherein like numbers reference like elements.

Fig. 1 is a view illustrating an example of a configuration of a heat flow measuring apparatus.
Fig. 2 is a radial section view of the heat flow measuring apparatus.
Fig. 3 is an axial section view of the heat flow measuring apparatus.
Fig. 4 is a side view of one end of the heat flow measuring apparatus when viewed from the axis side.
Fig. 5 is a side view of the other end of the heat flow measuring apparatus when viewed from the axis side.
Fig. 6 is a sectional view in a radial direction for depicting how to transfer heat in the heat ring.
Fig. 7 is a sectional view in a radial direction for depicting how to transfer heat in the heat ring.
Fig. 8 is a sectional view in a radial direction for depicting how to transfer heat in the heat ring.
Fig. 9 is a graph illustrating an example of a relationship between heat flow and a metabolic rate.
Fig. 10 is a sectional view illustrating a modification example of the heat ring.
Fig. 11 is a perspective view illustrating a modification example of the metabolism measuring apparatus.

## DESCRIPTION OF EXEMPLARY EMBODIMENTS

First Embodiment

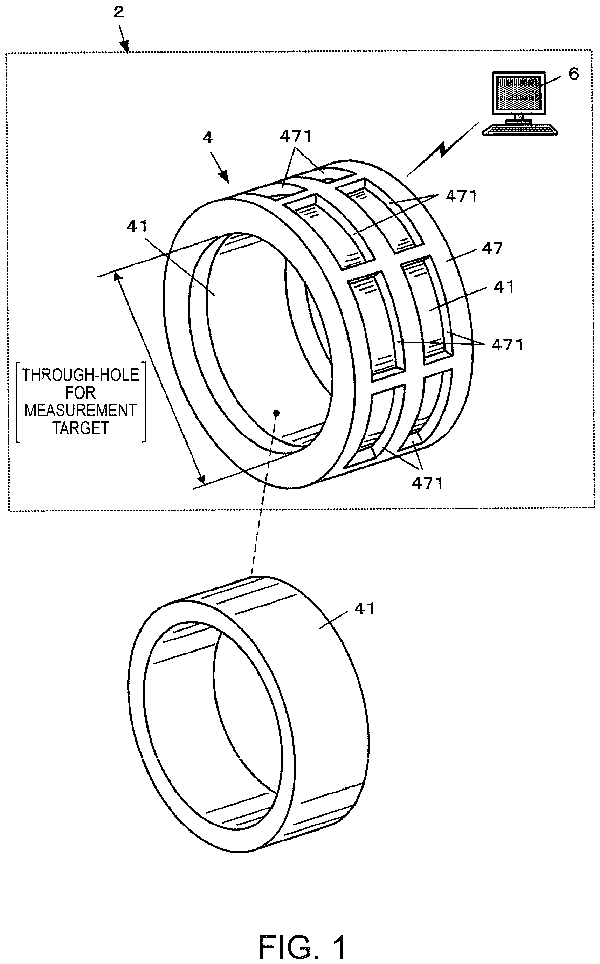

Fig. 1 illustrates an example of a configuration of a metabolism measuring apparatus according to the present embodiment. A metabolism measuring apparatus 2 includes a ring-shaped heat flow measuring apparatus 4 worn on a finger as a measurement target and a computing apparatus 6 that computes a metabolic rate using estimation based on a temperature measured by the heat flow measuring apparatus 4 or heat flow Q.

The computing apparatus 6 corresponds to a computer which includes a computing unit, a storage unit, a communication unit, an input unit, and an image displaying unit and which can execute a program by which the metabolic rate is computed. The computing apparatus 6 can be realized by dedicated hardware; however, may also be realized by a general-purpose personal computer, a smart phone, a tablet type computer, a wearable computer such as a wrist watch type, or the like by executing an application program which performs data communication with the heat flow measuring apparatus 4 and computing of metabolic rate.

The heat flow measuring apparatus 4 is a wearable computer which means an electronic apparatus having 1) a continuous temperature measuring function, 2) a computing function of calculating living body information such as the heat flow Q using the measured temperature, 3) a data logger function of storing the measured value and the calculated living body information, and 4) a data communicating function of transmitting the stored data to the computing apparatus 6.

Specifically, the heat flow measuring apparatus 4 has an annular body which is worn on an outer circumference of a measurement target which contains a heat source, includes a heat ring 41 and a protective section 47 that covers both end surfaces and the outer circumference of the heat ring 41, and has a wide ring-like appearance.

The heat ring 41 corresponds to an apparatus that surrounds the measurement target (finger of a person in the present embodiment), receives heat from the contact portion on the inner circumference, and naturally releases heat to the atmosphere from the outer circumference. Each temperature on the radially inner side (upstream side in the transfer) and on the radially outer side (downstream side in the transfer) can be measured and it is possible to obtain the heat flow Q based on the measured value. The inner diameter of the heat ring 41 can be appropriately set depending on the external appearance of the assumed measurement target. According to the present embodiment, a finger of a person is assumed as the measurement target such that the ring is formed as an ellipse including a circle. Various sizes are prepared for the inner diameter thereof and the diameter is substantially 10 mm to 30 mm.

The protective section 47 is molded using a synthetic resin such as an acrylonitrile butadiene styrene copolymer (ABS) resin or a poly methyl methacrylate (PMMA) resin, prevents the object other than the measurement target from coming into contact with the heat ring 41, and contains a controlling electronic component (to be described below) of the heat flow measuring apparatus 4 or the like. In terms of heat insulation, it is desirable to use a material which has thermal conductivity $\lambda p < 0.3$ (W/ (m·K)). It is preferable that the heat ring 41 is covered with a portion having a significant thickness in terms of the heat insulation; however, the thickness is set to about 1 mm to 5 mm in terms of a weight increase and wearability.

One or a plurality of appropriate outer circumferential openings 471 are provided on the portion which covers the outer circumferential surface of the heat ring 41 among the protective section 47 and the heat ring 41 is in contact with surroundings and the natural release of heat is improved. An opening ratio is appropriately set taking into account the effects of preventing contact with another object and heat releasability of the heat ring 41. For example, the opening ratio is set to 30% to 90%. Shapes or the distribution of the openings are not limited to one but the opening ratio may be lowered on the side portion with which an adjacent finger is likely to come into contact, and the upper portion and the lower portion may have a high opening ratio such that openings may be unevenly formed to have a high opening ratio on the upper and lower portions.

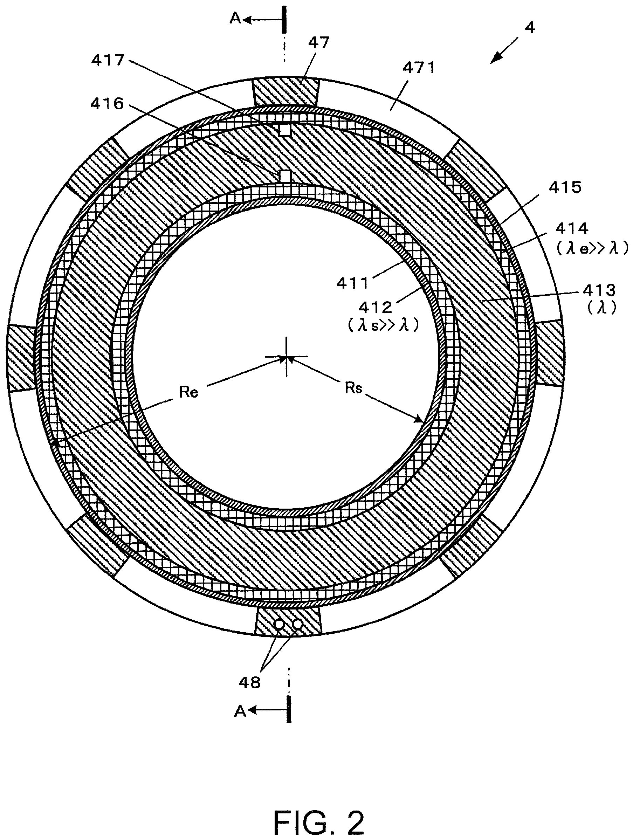

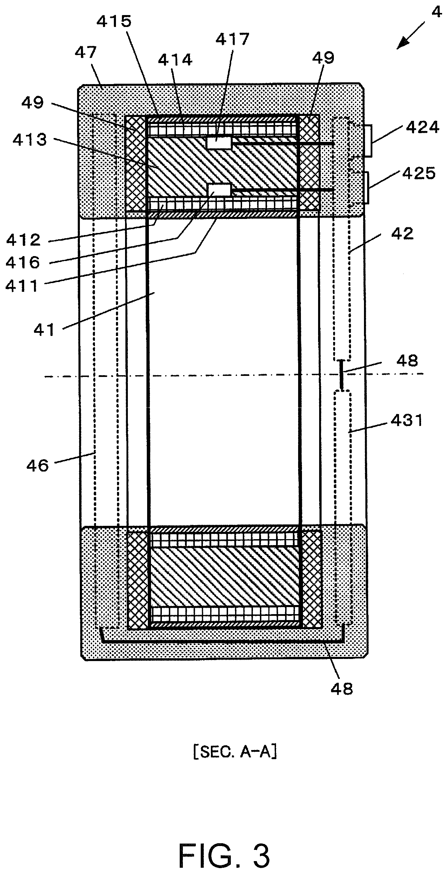

Fig. 2 and Fig. 3 are a sectional view in the radial direction and a sectional view in the axial direction of the heat flow measuring apparatus 4, respectively.

The heat ring 41 has a layer structure in the radial direction and includes, from the inner side, a heat receiving section 411, a first heat diffusing member 412, a heat transfer layer 413, a second heat diffusing member 414, a heat releasing section 415. The heat ring 41 also has a first temperature sensor 416 that measures a temperature of the first heat diffusing member 412, and a second temperature sensor 417 that measures a temperature of the second heat diffusing member 414 .

The heat receiving section 411 comes into contact with the measurement target and receives heat. According to the present embodiment, the heat receiving section 411 is realized by a Teflon (registered trademark) thin film layer in terms of protecting the surface and an inner surface of the first heat diffusing member 412 may also function as the heat receiving section 411.

The first heat diffusing member 412 is thermally connected to the heat receiving section 411 and is an annular member that diffuses heat transferred from the heat receiving section 411 in a circumferential direction of the heat ring 41. For example, the first heat diffusing member 412 is formed of a material with high thermal conductivity (thermal conductivity $\lambda s > 100$ (W/(m·K))), such as aluminum, copper, silver, and carbon nanotube.

A thickness d of the first heat diffusing member 412 in the radial direction can be set in terms of the thermal conductivity and mechanical strength and for example, thickness d=0.05 mm to 0.5 mm.

A width w of the first heat diffusing member 412 in the axial direction depends on a size of the assumed heat source. Since the finger is assumed in the present embodiment and an arterial blood vessel is assumed to have the diameter of maximum 10 mm as the heat source, at least half the diameter is assumed in terms of promoting equalization of the temperature distribution. Then, the width w is set to about 5 mm to 10 mm, taking into account a distance between knuckles of the finger.

The heat transfer layer 413 is interposed between the outer circumferential surface (first curved surface) of the first heat diffusing member 412 and the inner circumferential surface (second curved surface) of the second heat diffusing member 414 and a thermally connected heat transfer material transfers heat diffused over the entire circumference in the first heat diffusing member 412 to the second heat diffusing member 414.

A material of the heat transfer layer 413 is selected such that the thermal conductivity $\lambda$ is less than the thermal conductivity $\lambda s$ of the first heat diffusing member and such that the heat is transferred as uniformly as possible from the first heat diffusing member 412 to the second heat diffusing member 414. For example, when the first heat diffusing member has the thermal conductivity $\lambda s > 100$ (W/(m·K)), the thermal conductivity $\lambda$ of the heat transfer layer 413 is from 0.3 (W/(m·K)) to 100 (W/(m·K)) and thus it is possible to select from materials such as glass, ceramic, metal such as stainless steel or titanium, plastic, a synthetic material which is formed by metal power or a carbon material (carbon nanotube, or the like) dispersed in glass or a resin, or the like. The material of the heat transfer layer 413 includes glass, ceramic, metal such as stainless steel or titanium, plastic, or the like and it is preferable that the thermal conductivity $\lambda$ is 0.3 (W/(m·K)) to 20 (W/(m·K)).

The thicker the thickness of the heat transfer layer 413 is, the more likely the temperature difference between the inner and outer sides of the heat ring 41 is to be generated, such that it is advantageous to measure the heat flow with high accuracy. However, since the heat flow measuring apparatus 4 of the present embodiment is assumed to be worn on a finger, the thickness is set to about 1 mm to 5 mm taking into account weight or wearability, or interference from other fingers or clothing.

The second heat diffusing member 414 causes the heat transferred from the heat transfer layer 413 to further diffuse in the circumferential direction of the heat ring 41 and to be equalized. The second heat diffusing member 414 is formed of a material which has a thermal conductivity $\lambda e$ which is higher than the thermal conductivity $\lambda$ of the heat transfer layer 413. The same material as the first heat diffusing member 412 may be selected.

The heat releasing section 415 releases heat transferred from the second heat diffusing member 414 to the surroundings. In the present embodiment, the heat releasing section 415 is realized by a Teflon thin film layer in terms of a surface protection; however, the outer circumferential surface of the second heat diffusing member 414 may function as the heat releasing section 415 also.

The first temperature sensor 416 and the second temperature sensor 417 are realized using a known temperature measurement sensor such as a thermistor or a thermocouple and are connected to a CPU module 42.

An insulation material 49 is provided to be bonded on one end surface of the heat ring 41 in the axial direction and thus, the transferring of the heat to the protective section 47 from a surface in the axial direction is suppressed.

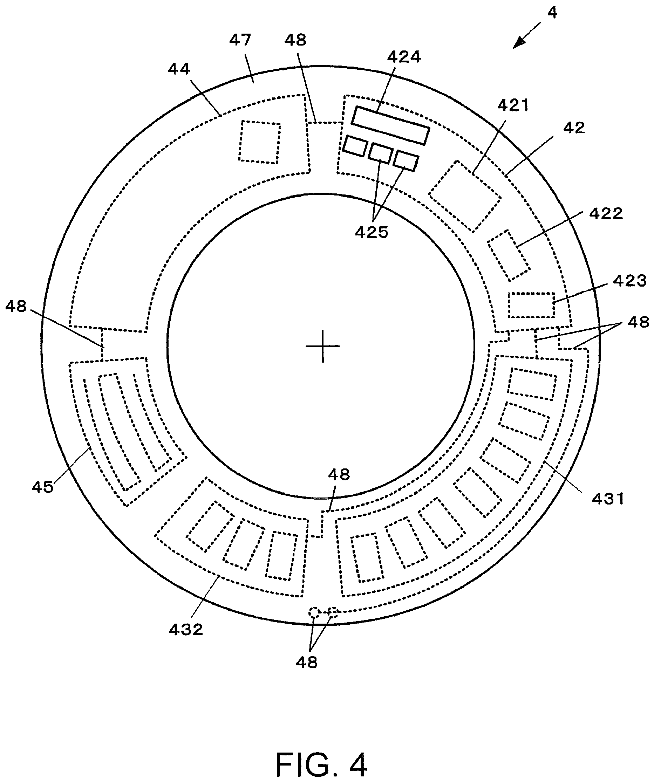

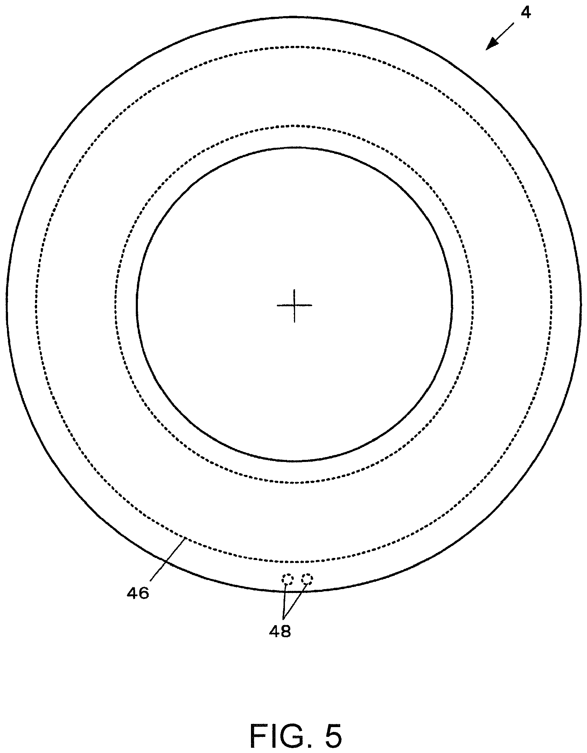

Fig. 4 and Fig. 5 are one end side view and the other end side view of the heat flow measuring apparatus 4 when viewed from the axial side. The protective section 47 contains a controlling electronic component or the like (to be described below) of the heat flow measuring apparatus 4 at a portion which covers the end surface of the heat ring 41 in the axial direction.

Specifically, the protective section contains the central processing unit (CPU) module 42, a main memory module 431, an analysis data memory module 432, a wireless communication module 44, and an antenna module 45. In addition, a battery 46 is contained to supply power to the modules. A wire 48 for associated data communication or power supply is also appropriately contained in the protective section 47. The battery 46 preferably has a rechargeable configuration through non-contact or wireless charge.

The CPU module 42 corresponds to a control board that controls the heat flow measuring apparatus 4. For example, a CPU 421 or an IC memory 422, an interface circuit 423, an operation switch 424, and a status displaying LED 425 are mounted in the CPU module 42. It is needless to say that a configuration which is realized through integration of a part or all of the components into one integrated circuit can be employed.

Thus, the CPU module 42 causes the CPU 421 to execute a program stored in the IC memory 422 and collectively controls the heat flow measuring apparatus 4.

Specifically, the CPU module 42 realizes the following functions.

1) a timekeeping function using a system clock of the CPU 421

2) a measuring function of cyclically acquiring a temperature Ts of the first heat diffusing member 412 and a temperature Te of the second heat diffusing member 414 from the first temperature sensor 416 and the second temperature sensor 417, respectively, which are connected to each other through the interface circuit 423

3) a computing function of calculating heat flow Q and heat flux q of heat flowing through the heat ring 41 per unit time with the measured temperature Ts and temperature Te using the main memory module 431

4) a data logger function of storing the temperature Ts, the temperature Te, the heat flow Q, and the like, in time series, to the analysis data memory module 432

5) a data communicating function of realizing data communication with the computing apparatus 6 through the wireless communication module 44 and the antenna module 45 and transmitting analysis data which is stored in the analysis data memory module 432

It is needless to say that other functions may be appropriately realized.

Description of Operation

Next, an operation of the metabolism measuring apparatus 2 will be described.

First, the heat flow measuring apparatus 4 is worn on the measurement target and power is applied through operating the operation switch 424. In the present embodiment, the heat flow measuring apparatus 4 is fit on a finger of a measurement target subject and skin of the finger is substantially in full contact with the inner circumferential surface of the heat ring 41.

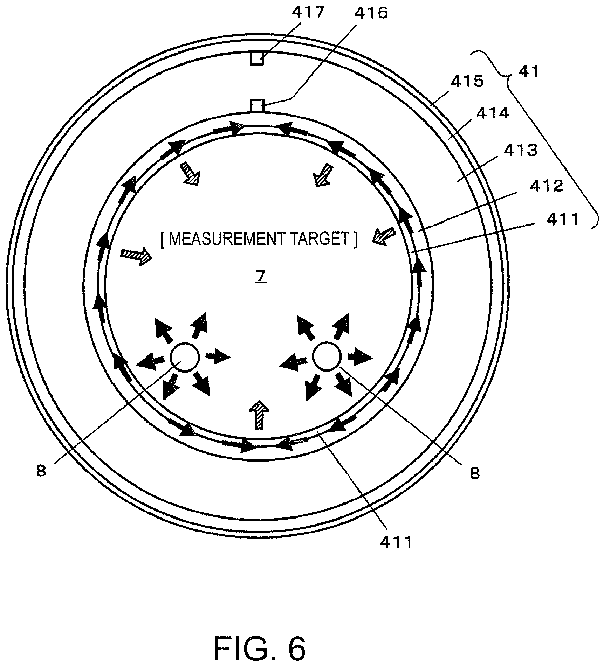

Fig. 6 to Fig. 8 are sectional views in the radial direction for depicting how the heat is transferred in the heat ring 41 and show movement of the heat by an arrow. For easy understanding, hatching of sectional surfaces is omitted.

As illustrated in Fig. 6, when a finger of a person is set as a measurement target 7, the heat source is an arterial blood vessel 8 and heat flow is dispersed from the arterial blood vessel 8 in every direction (bold black arrows around the arterial blood vessel 8).

At this time, since the arterial blood vessel 8 is close to the palm side of the finger (lower side in Fig. 6), the heat first reaches a portion on the heat receiving section 411 of the heat ring 41, which is in contact with the palm side of the finger. In contrast, since the arterial blood vessel 8 is farthest from the dorsum of the finger, heat last reaches a portion on the heat receiving section 411, which is in contact with dorsum (upper side in Fig. 6) of the finger. That is, distribution of heat from the heat receiving section 411 can be irregular.

However, even when the distribution of the heat imparted from the heat receiving section 411 is irregular, the heat is diffused almost uniformly to the entire first heat diffusing member 412 (small black arrows in the circumferential direction of the heat receiving section 411 and the first heat diffusing member 412) such that there is no irregular distribution in the transferred heat, because the first heat diffusing member 412 to which the heat is transferred from the heat receiving section 411 has high thermal conductivity $\lambda$s. In other words, the first heat diffusing member 412 obtains the same temperature from the entire body regardless of a position of the heat source due to the heat diffusing effect. Normally, the temperature measured by the first temperature sensor 416 is not also influenced by the position of the heat source. In other words, even when one first temperature sensor 416 is provided, it is said that there is no influence on the measurement regardless of which orientation the heat flow measuring apparatus 4 has to be fitted to the finger. Reduction in the number of the temperature sensors significantly contributes to reduction in manufacturing costs and arbitrarily selectable wearing orientations enhance convenience in use.

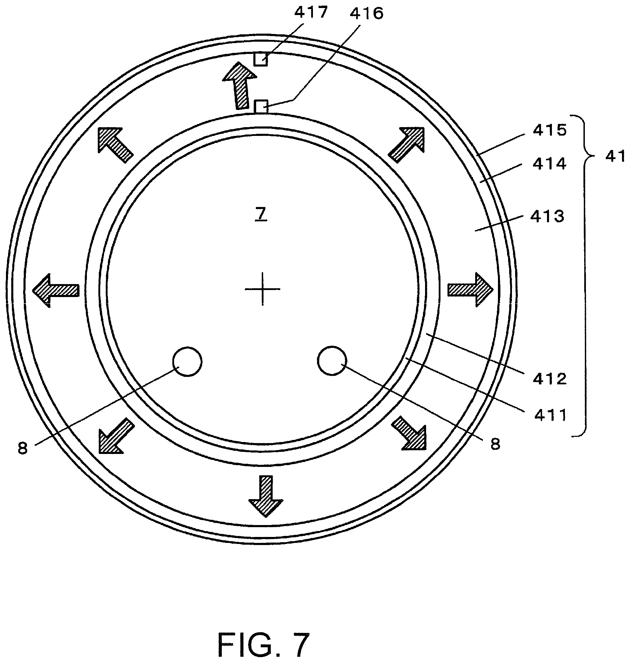

When the first heat diffusing member 412 has a uniform temperature, as illustrated in Fig. 7, heat is transferred from the first heat diffusing member 412 to the heat transfer layer 413 in the radial direction and reaches the second heat diffusing member 414 before long (hatched arrows radially arranged in the heat transfer layer 413). Further, the heat is transferred to the heat releasing section 415 and is released to the surroundings (atmosphere in the present embodiment) from an exposed portion as the outer circumferential opening 471.

The outer circumferential openings 471 do not have to be arranged uniformly over the entire circumference of the protective section 47. At first glance, it is considered that temperature irregularity will occur because a portion of the heat releasing section 415 which is covered by the protective section 47 and a portion which is exposed by the outer circumferential opening 471 have different degrees of releasing heat from each other; however, this is not a problem because the second heat diffusing member 414 has a high terminal conductivity λe. Therefore, the temperature measured by the second temperature sensor 417 does not depend on a relative position to the outer circumferential openings 471. In this manner, when the heat flow measuring apparatus 4 is manufactured as thin as possible, design of the outer circumferential openings 471 or a layout of the second temperature sensor 417 is determined in a significantly flexible way and thus, effects that increase room for enhancing the wearability or usability are achieved.

When the power of the heat flow measuring apparatus 4 is ON, the CPU 421 reads and executes a program from the IC memory 422 in the CPU module 42 and a timekeeping process of measurement time using a system clock and a continuous measuring process of the temperature Ts by the first temperature sensor 416 and the temperature Te by the second temperature sensor 417 are started.

When an appropriate period of time elapses after the measurement start to the extent that a temperature can be stably measured by the second temperature sensor 417, the CPU module 42 calculates the heat flow Q and a deep temperature Tc through a following method.

First, heat flux q which is transferred to the heat ring in the radial direction is represented by Equation (1). Here, λ= thermal conductivity of the heat transfer layer 413, and dT/dr= a temperature gradient of the heat ring 41 in the radial direction.

$$q = -\lambda \frac{dT}{dr} \qquad (1)$$

Here, the heat flux q at a position r of the heat ring 41 in the radial direction has a relationship of Equation (2) with respect to the heat flow Q of heat flowing a surface of a cylinder by unit time.

$$q = \frac{Q}{2\pi r} \qquad (2)$$

A differentiated temperature gradient dT is represented by Equation (3) from Equation (1) and Equation (2).

$$dT = -\frac{Q}{2\pi r} \frac{dr}{r} \qquad (3)$$

When Equation (3) is integrated by r in the radial direction and the temperature T=Ts (temperature measured by the first temperature sensor 416) at a position r=Rs (a radius of the inner circumference of the heat receiving section 411: refer to Fig. 2) as a boundary condition, the temperature T at the position r in the radial direction is represented by Equation (4). Here, log means a natural logarithm.

$$T = -\frac{Q}{2\pi\lambda}\log r + Ts + \frac{Q}{2\pi\lambda}\log Rs \qquad (4)$$

In Equation (4), when the temperature T=Te (temperature measured by the second temperature sensor 417) at the position r=Re (a radius of the outer circumference of the heat releasing section 415: refer to Fig. 2), a temperature gradient within the heat ring 41 is represented by Equation (5).

$$Ts - Te = \frac{\log(Re/Rs)}{2\pi\lambda}Q \qquad (5)$$

Accordingly, using the temperature Ts measured by the first temperature sensor 416 and the temperature Te measured by the second temperature sensor 417 from Equation (5), the heat flow Q which is started from the measurement target can be calculated by Equation (6).

$$Q = \frac{2\pi r}{\log(Re/Rs)}(Ts - Te) \qquad (6)$$

When the heat flow Q is calculated, the CPU module 42 further calculates the deep temperature Tc of the measurement target. The deep temperature Tc means a temperature at a core portion which is unlikely to be influenced by a surrounding temperature, that is, a deep body temperature. In recent years, the deep body temperature is used as a parameter of various types of healthcare for achieving heat stroke prevention or sound sleep. In a measurement apparatus of the related art, which measures the deep temperature Tc using a temperature on the skin's surface, there is a need to separately provide an electronic heater unit for compensating (heat flow compensation) for heat released from the skin's surface; however, according to the present embodiment, the heat flow compensation is realized by a heat diffusing effect of the first heat diffusing member 412 and by using the structure in which the first heat diffusing member 412 is covered with the heat transfer layer 413 having low thermal conductivity λ and thereby, it is possible to measure the deep temperature Tc without providing the heater unit.

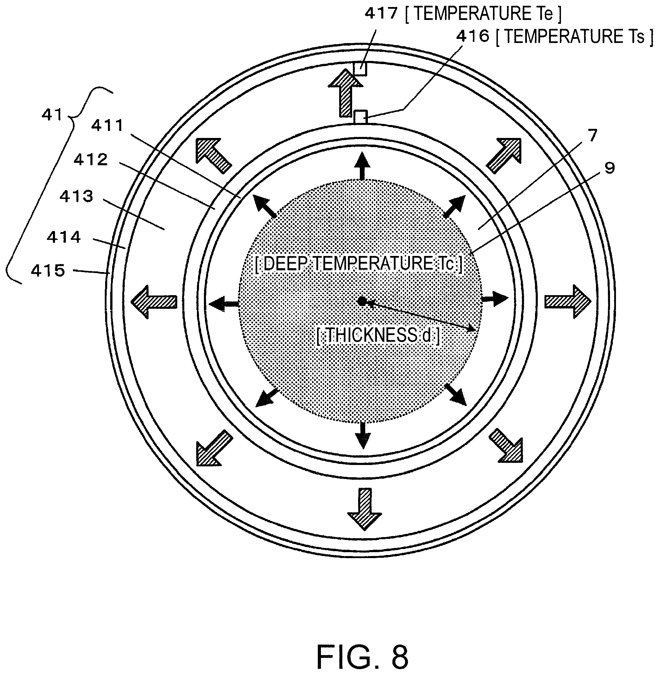

Specifically, as described above, the heat from the measurement target 7 regardless of the position of the heat source becomes uniform over the entire circumference due to the heat diffusing effect of the first heat diffusing member 412 (refer to Fig. 6). It is considered that the farther apart a portion (upper side in Fig. 6 and dorsum of the finger) of the measurement target 7 is from the heat source (arterial blood vessel 8), the lower the temperature is; however, the heat is transferred through the first heat diffusing member 412 (refer to hatched arrows depicted to have a central orientation above from the arterial blood vessel 8 in Fig. 6) and thereby, the temperature of a portion which is apart from the heat source and thus is likely to have a relatively low temperature is increased. Then, before long, as illustrated in Fig. 8, it is considered that a cylindrical heat source (deep layer 9) having the deep temperature Tc is present to a certain depth such that the heat is propagated in the outer circumferential direction of the heat ring 41 with a constant gradient.

When a temperature of the deepest portion, that is, a portion having the highest temperature, in the deep layer 9 is referred to as the deep temperature Tc, the deep layer 9 is considered as a transferring portion through which heat is transferred at thermal conductivity λt. Hence, when a thickness of the deep layer 9 is represented by d, a temperature difference between the deep temperature Tc and the temperature Ts of the heat receiving section 411 is represented by Equation (7), using Equation (5) above.

$$Tc - Ts = \frac{\log\left(\frac{Rs}{Rs - d}\right)}{2\pi\lambda t} Q \qquad (7)$$

In addition, using Equation (5) and Equation (7), a temperature difference between the deep temperature Tc and the temperature Te which is measured by the second temperature sensor 417 is represented by Equation (8).

$$\left. \begin{aligned} Tc - Te &= (Tc - Ts) + (Ts - Te) \\ &= \frac{\log\left(\frac{Rs}{Rs - d}\right)}{2\pi\lambda t} Q + \frac{\log(Re/Rs)}{2\pi\lambda} Q \end{aligned} \right\} \qquad (8)$$

Then, both sides of Equation (8) and Equation (7) are divided by the heat flow Q and the heat flow Q is removed. Then, when the result is referred to as D, Equation (9) is obtained.

$$D = \frac{(Tc - Ts)}{(Tc - Te)} = \frac{\dfrac{\log\left(\dfrac{Rs}{Rs - d}\right)}{\lambda t}}{\dfrac{\log\left(\dfrac{Rs}{Rs - d}\right)}{\lambda t} + \dfrac{\log\left(\dfrac{Re}{Rs}\right)}{\lambda}} \qquad (9)$$

Here, D can be regarded as a constant which does not depend on the heat flow Q from the measurement target 7. Accordingly, it is possible to calculate the deep temperature Tc using Equation (10).

$$Tc = \frac{Ts - DTe}{1 - D} \qquad (10)$$

The CPU module 42 calculates the heat flow Q using Equation (6) whenever the temperature Ts and the temperature Te are measured. Then, an elapsing time from the measurement start, the temperature Ts, the temperature Te, the heat flow Q, and the deep temperature Tc are associated and are stored in time series in the analysis data memory module 432 by storage control by the CPU module 42. In parallel with the storage control, the CPU module 42 may establish data communication with the computation apparatus 6 and may perform control of transmission of data stored in the analysis data memory module 432.

The computation apparatus 6 obtains a metabolic rate Qall from the heat flow Q contained in data received from the heat flow measuring apparatus 4 and displays the calculating results sequentially. Known statistical processing such as graph display may be performed.

Specifically, a correlation function F between the heat flow Q and the metabolic rate Qall, which is obtained in advance in experiments, is stored in the computation apparatus 6 such that the metabolic rate Qall is obtained with reference to the correlation function.

The function F can be prepared using, for example, profile data, as follows.

When a person is assumed as the measurement target, an amount of inhaled oxygen VO2 and an amount of exhaled carbon dioxide VCO2 are measured by an exhaled gas analyzer for many subjects who have different attribute parameters (profile) of personal information such as gender, stature, weight, age, or body fat, in a state in which additional conditions of exercise or temperature environment are changed, and the metabolic rate Qall is calculated by Equation (11). In addition, at the same time, the heat flow Q is measured by the heat flow measuring apparatus 4.

$$Q\,all = 4.686 + \frac{\dfrac{VCO2}{VO2} - 0.707}{0.293} \times 0.361 \qquad (11)$$

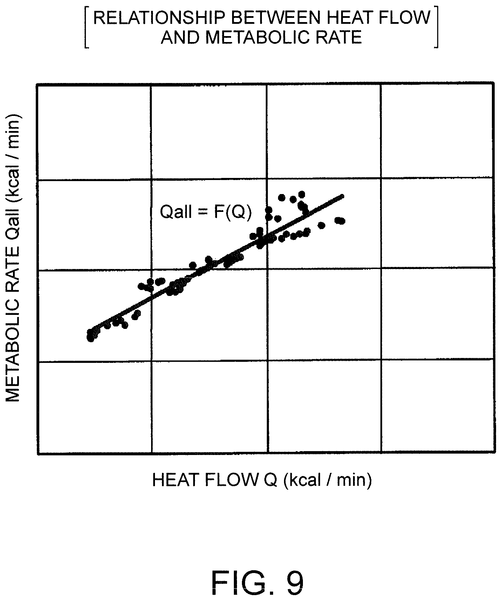

A correlation between the heat flow Q and the metabolic rate Qall is obtained for each group classified depending on parameters such as gender, stature, weight, age, and body fat, the measurement data is subject to regression analysis, and thereby, the function F is obtained (refer to Fig. 9). The conditions of the parameters which define a group and the obtained function F are associated with each other and are stored in the computation apparatus 6 for each group. Alternatively, the measurement data may be included in a program.

An operator of the metabolism measuring apparatus 2 inputs a value of the parameters of the measurement target in the computation apparatus 6. The computation apparatus 6 determines a group suitable for the input value. With reference to the function F corresponding to the determined group, a metabolic rate Qall is obtained using the heat flow Q received from the heat flow measuring apparatus 4. Values of the heat flow Q and the metabolic rate Qall are numerically displayed or displayed into a graph in time series.

As above, according to the present embodiment, even when positional irregularity occurs in transferring heat to the heat receiving section 411 from the measurement target 7, a uniform temperature is obtained due to the heat diffusing effect of the first heat diffusing member 412. Therefore, regardless of a positional relationship between the heat source (arterial blood vessel 8) and the first temperature sensor 416, it is possible to measure the temperature Ts with stability and high accuracy. In addition, since the first temperature sensor 416 can be configured of only one temperature sensor, it is possible to suppress the manufacturing costs to be lowered.

In addition, when heat releasing is focused, irregular heat releasing occurs because the heat releasing section 415 releases heat to the atmosphere from a portion which is exposed through the outer circumferential openings 471; however, regardless of the irregular heat releasing, it is possible to measure the temperature Te by the second temperature sensor 417 due to the heat diffusing effect of the second heat diffusing member 414 with stability and high accuracy.

In addition, in this configuration, the thermal conductivity of the first heat diffusing member 412 and the second heat diffusing member 414 is higher than the thermal conductivity of the heat transfer layer 413 and the maximum temperature gradient of the former is higher than that of the latter. In this manner, heat is transferred from the first heat diffusing member 412 to the second heat diffusing member 414 and it is possible to reduce a period of time taken for the temperature to become uniform. Accordingly, irregular heat transferring from the first heat diffusing member 412 to the second heat diffusing member 414 is prevented and still further, in this structure, the relative positional relationship between the heat source and the temperature sensor is unlikely to be influenced by the irregularity.

In addition, since the heat flow measuring apparatus 4 has a ring shape with which there is no need to concern about the wearing orientation to a finger (measurement target), the heat flow measuring apparatus 4 is easily worn and has a structure suitable for monitoring for a long period of time.

Modification Examples

The embodiments of the invention are not limited to above, and it is possible to appropriately perform addition, omitting, and modification of a component.

Modification Example 1

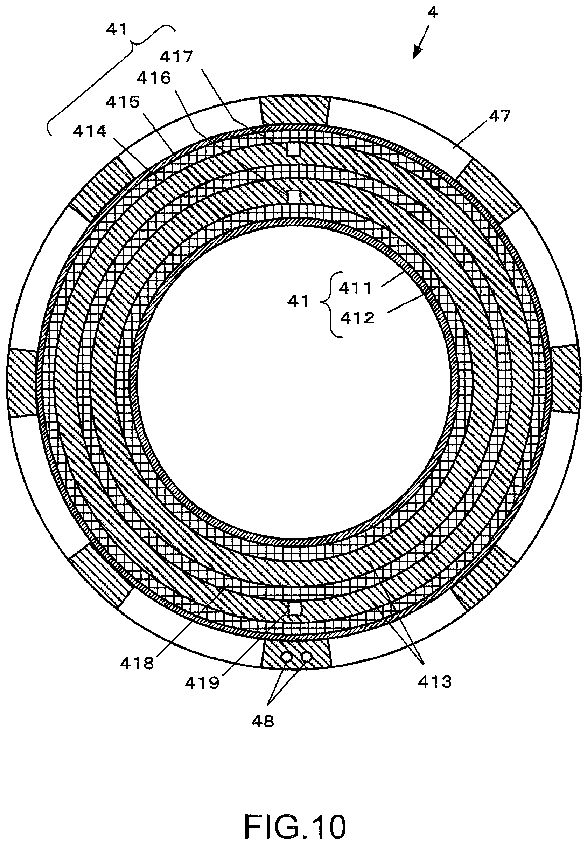

For example, as illustrated in Fig. 10, it is possible to employ a configuration in which a third heat diffusing member 418 and a third temperature sensor 419 that measures a temperature of the third heat diffusing member 418 are added to the heat transfer layer 413. The third heat diffusing member 418 is realized similar to the first heat diffusing member 412 and the second heat diffusing member 414. In addition, the third temperature sensor 419 is realized similar to the first temperature sensor 416 or the second temperature sensor 417. According to the configuration, since it is possible to further enhance heat diffusing performance in the circumferential direction of the heat ring 41 than in the embodiments described above, it is possible to realize measurement without an influence of the relative positions of the heat source and the temperature sensor and the relative positions of a cooling source and a temperature sensor.

In addition, in a case where the configuration is applied, the heat flow Q may be calculated using the temperature measured by the third temperature sensor 419 instead of the temperature Te measured by the second temperature sensor 417 in Equation (6) or the heat flow Q may be calculated using the temperature measured by the third temperature sensor 419 instead of the temperature Ts measured by the first temperature sensor 416 in Equation (6).

Further, it is possible to employ a configuration in which a combination of the temperature sensors applied to Equation (6) is formed by selecting a plurality of sensors from the first temperature sensor 416, the second temperature sensor 417, the third temperature sensor 419 and the heat flow Q is primarily calculated, and then, an average of calculated results becomes the final heat flow Q.

Modification Example 2

According to the present embodiment described above, the description is provided on a premise that a finger of a person is set as the measurement target; however, when an outer diameter of the measurement target is about the size of the finger, it is needless to say that the measurement may be performed on a toe or the like.

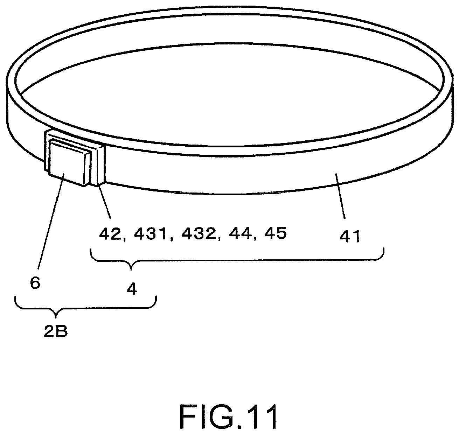

In addition, for a person, the measurement target may not be limited to extremities such as a finger but may be the trunk of a body (for example, thorax or abdomen). In this case, for example, as illustrated by a metabolism measuring apparatus 2B in Fig. 11, the heat ring 41 of the heat flow measuring apparatus 4 may have a diameter greater than that in the embodiments described above. Specifically, when the heat ring 41 is a belt type having a width of about 10 mm to 100 mm in the axial direction and a perimeter of about 600 mm to 1200 mm, the belt type heat ring is suitable because wearability is achieved and it is possible to have as small an influence of heat outflow as possible from a side surface. In a portion corresponding to a buckle of a belt, the CPU module 42 or the main memory module 431, the analysis data memory module 432, the wireless communicating module 44, the antenna module 45, or the like may be installed and further, a small computation apparatus 6 may be provided removably. In the case of the belt type, the protective section 47 can be omitted.

Modification Example 3

In addition, in the configuration, according to the present embodiment described above, the heat flow Q is calculated using the temperature Ts and the temperature Te by the heat flow measuring apparatus 4; however, a configuration may be employed, in which the heat flow measuring apparatus 4 functions only as a temperature measuring and storing apparatus which measures the temperature Ts and the temperature Te and stores the temperatures in time series and the heat flow Q is calculated by the computation apparatus 6.

Modification Example 4

In addition, in the present embodiment described above, a living body represented by a person is the measurement target; however, the measurement target is not limited thereto. For example, an industrial pipe or tube, a tank, a heat insulating case, a furnace, or the like for which a temperature control or the like is needed may be set as the measurement target.

**Claims**

1. A heat flow measuring apparatus (4) comprising:

   an annular first heat diffusing member (412);
   a first temperature sensor (416) that is thermally connected to the first heat diffusing member (412); an annular second heat diffusing member (414);
   a second temperature sensor (417) that is thermally connected to the second heat diffusing member (414); and
   a heat transfer layer (413) that is disposed between the first heat diffusing member (412) and the second heat diffusing member (414),
   wherein the thermal conductivity ($\lambda_S$) of the first heat diffusing member (412) is higher than the thermal conductivity ($\lambda$) of the heat transfer layer (413), and
   wherein the thermal conductivity ($\lambda_E$) of the second heat diffusing member (414) is higher than the thermal conductivity ($\lambda$) of the heat transfer layer (413).

2. The heat flow measuring apparatus (4) according to claim 1,
   wherein the thermal conductivity of the first heat diffusing member and the thermal conductivity ($\lambda_E$) of the second heat diffusing member (414) are 100 (W/(m·K)) or more and the thermal conductivity ($\lambda$) of the heat transfer layer (413) is 0.3 (W/(m·K)) to 100 (W/(m·K)).

3. The heat flow measuring apparatus (4) according to one of the preceding claims,
   wherein the first heat diffusing member (412) has a heat receiving area which is to be in contact with a measurement target and is 204 (mm$^2$) to 690 (mm$^2$) .

4. The heat flow measuring apparatus (4) according to one of the preceding claims,
   wherein the maximum temperature gradient of the first heat diffusing member (412) is larger than the maximum temperature gradient of the heat transfer layer (413) and the maximum temperature gradient of the second heat diffusing member (414) is larger than the maximum temperature gradient of the heat transfer layer(413).

5. The heat flow measuring apparatus (4) according to one of the preceding claims, further comprising:

   the heat transfer layer comprising a first heat transfer layer and a second heat transfer layer;
   a third heat diffusing member is interposed between the first heat diffusing member and the second heat diffusing member;
   a third temperature sensor that is provided for measuring a temperature of the third heat diffusing member;
   wherein thermal conductivity of the third heat diffusing member is higher than thermal conductivity of the heat transfer layer.

6. The heat flow measuring apparatus according to claim 5,
   the thermal conductivity of the third heat diffusing member are 100 (W/(m·K)) or more.

7. The heat flow measuring apparatus according to one of the preceding claims,
   a heat flow is calculated using the two or three temperatures among the temperature of the first heat diffusing member, the temperature of the second heat diffusing member and the temperature of the third heat diffusing member.

8. The heat flow measuring apparatus (4) according to one of the preceding claims,
   wherein a protective section (47) that has an opening is provided to cover the outer circumference of the second diffusing member.

9. The heat flow measuring apparatus (4) according to claim 8,
   wherein thermal conductivity of the protective section (47) is lower than the thermal conductivity of the heat transfer layer.

10. A metabolism measuring apparatus (2) comprising:

    the heat flow measuring apparatus (4) according to one of the preceding claims; and
    a computing apparatus (6) that estimates a metabolic rate.

**11.** A deep temperature measuring apparatus comprising
the heat flow measuring apparatus according to one of claims 1 to 9.

**Patentansprüche**

**1.** Wärmeflussmessvorrichtung (4) umfassend:

ein ringförmiges erstes Wärmeverteilungselement (412);
einen ersten Temperatursensor (416), der thermisch mit dem ersten Wärmeverteilungselement (412) verbunden ist;
ein ringförmiges zweites Wärmeverteilungselement (414);
einen zweiten Temperatursensor (417), der thermisch mit dem zweiten Wärmeverteilungselement (414) verbunden ist; und
eine Wärmeübertragungsschicht (413), die zwischen dem ersten Wärmeverteilungselement (412) und dem zweiten Wärmeverteilungselement (414) angeordnet ist,
wobei die thermische Leitfähigkeit ($\lambda_S$) des ersten Wärmeverteilungselements (412) höher ist als die thermische Leitfähigkeit ($\lambda$) der Wärmeübertragungsschicht (413), und
wobei die thermische Leitfähigkeit ($\lambda_E$) des zweiten Wärmeverteilungselements (414) höher ist als die thermische Leitfähigkeit ($\lambda$) der Wärmeübertragungsschicht (413).

**2.** Wärmeflussmessvorrichtung (4) nach Anspruch 1,
wobei die thermische Leitfähigkeit des ersten Wärmeverteilungselements und die thermische Leitfähigkeit ($\lambda_E$) des zweiten Wärmeverteilungselements (414) 100 (W/(m·K)) oder mehr betragen, und die thermische Leitfähigkeit ($\lambda$) der Wärmeübertragungsschicht (413) 0,3 (W/(m·K)) bis 100 (W/(m·K)) beträgt.

**3.** Wärmeflussmessvorrichtung (4) nach einem der vorstehenden Ansprüche,
wobei das erste Wärmeverteilungselement (412) eine Wärmeaufnahmefläche aufweist, die mit einem Messziel in Kontakt stehen muss und 204 ($mm^2$) bis 690 ($mm^2$) beträgt.

**4.** Wärmeflussmessvorrichtung (4) nach einem der vorstehenden Ansprüche,
wobei der maximale Temperaturgradient des ersten Wärmeverteilungselements (412) größer ist als der maximale Temperaturgradient der Wärmeübertragungsschicht (413) und der maximale Temperaturgradient des zweiten Wärmeverteilungselements (414) größer ist als der maximale Temperaturgradient der Wärmeübertragungsschicht (413).

**5.** Wärmeflussmessvorrichtung (4) nach einem der vorstehenden Ansprüche, weiter umfassend:

die Wärmeübertragungsschicht, umfassend eine erste Wärmeübertragungsschicht und eine zweite Wärmeübertragungsschicht;
ein drittes Wärmeverteilungselement ist zwischen dem ersten Wärmeverteilungselement und dem zweiten Wärmeverteilungselement eingefügt;
einen dritten Temperatursensor, der zum Messen einer Temperatur des dritten Wärmeverteilungselements bereitgestellt ist;
wobei die thermische Leitfähigkeit des dritten Wärmeverteilungselements höher ist als die thermische Leitfähigkeit der Wärmeübertragungsschicht.

**6.** Wärmeflussmessvorrichtung nach Anspruch 5,
die thermische Leitfähigkeit des dritten Wärmeverteilungselements beträgt 100 (W / (m ·K)) oder mehr.

**7.** Wärmeflussmessvorrichtung nach einem der vorstehenden Ansprüche,
ein Wärmefluss wird unter Verwendung der zwei oder drei Temperaturen unter der Temperatur des ersten Wärmeverteilungselements, der Temperatur des zweiten Wärmeverteilungselements und der Temperatur des dritten Wärmeverteilungselements berechnet.

**8.** Wärmeflussmessvorrichtung (4) nach einem der vorstehenden Ansprüche,
wobei ein Schutzabschnitt (47), der eine Öffnung aufweist, bereitgestellt ist, um den Außenumfang des zweiten Verteilerelements abzudecken.

**9.** Wärmeflussmessvorrichtung (4) nach Anspruch 8,
wobei die thermische Leitfähigkeit des Schutzabschnitts (47) niedriger ist als die thermische Leitfähigkeit der Wärmeübertragungsschicht.

**10.** Stoffwechselmessvorrichtung (2), umfassend:

die Wärmeflussmessvorrichtung (4) nach einem der vorstehenden Ansprüche; und
eine Computervorrichtung (6), die eine Stoffwechselrate schätzt.

**11.** Vorrichtung zur Tieftemperaturmessung, umfassend
die Wärmeflussmessvorrichtung nach einem der Ansprüche 1 bis 9.


**Revendications**

**1.** Appareil de mesure de flux thermique (4) comprenant :

un premier élément de diffusion de chaleur annulaire (412) ;
un premier capteur de température (416) qui est connecté thermiquement au premier élément de diffusion de chaleur (412) ;
un deuxième élément de diffusion de chaleur annulaire (414) ;
un deuxième capteur de température (417) qui est connecté thermiquement au deuxième élément de diffusion de chaleur (414) ; et
une couche de transfert de chaleur (413) qui est disposée entre le premier élément de diffusion de chaleur (412) et le deuxième élément de diffusion de chaleur (414),
dans lequel la conductibilité thermique ($\lambda_S$) du premier élément de diffusion de chaleur (412) est supérieure à la conductibilité thermique ($\lambda$) de la couche de transfert de chaleur (413), et
dans lequel la conductibilité thermique ($\lambda_E$) du deuxième élément de diffusion de chaleur (414) est supérieure à la conductibilité thermique ($\lambda$) de la couche de transfert de chaleur (413).

**2.** Appareil de mesure de flux thermique (4) selon la revendication 1,
dans lequel la conductibilité thermique du premier élément de diffusion de chaleur et la conductibilité thermique ($\lambda_E$) du deuxième élément de diffusion de chaleur (414) sont de 100 (W/(m-K)) ou plus et la conductibilité thermique ($\lambda$) de la couche de transfert de chaleur (413) est de 0,3 (W/(m-K)) à 100 (W/(m·K)).

**3.** Appareil de mesure de flux thermique (4) selon l'une quelconque des revendications précédentes,
dans lequel le premier élément de diffusion de chaleur (412) a une surface réceptrice de chaleur qui doit être en contact avec une cible de mesure et couvre 204 (mm$^2$) à 690 (mm$^2$).

**4.** Appareil de mesure de flux thermique (4) selon l'une quelconque des revendications précédentes,
dans lequel le gradient de température maximal du premier élément de diffusion de chaleur (412) est plus grand que le gradient de température maximal de la couche de transfert de chaleur (413) et le gradient de température maximal du deuxième élément de diffusion de chaleur (414) est plus grand que le gradient de température maximal de la couche de transfert de chaleur (413).

**5.** Appareil de mesure de flux thermique (4) selon l'une quelconque des revendications précédentes, comprenant en outre :

la couche de transfert de chaleur comprenant une première couche de transfert de chaleur et une deuxième couche de transfert de chaleur ;
un troisième élément de diffusion de chaleur est intercalé entre le premier élément de diffusion de chaleur et le deuxième élément de diffusion de chaleur ;
un troisième capteur de température qui est fourni pour mesurer une température du troisième élément de diffusion de chaleur ;
dans lequel la conductibilité thermique du troisième élément de diffusion de chaleur est supérieure à la conductibilité thermique de la couche de transfert de chaleur.

**6.** Appareil de mesure de flux thermique selon la revendication 5,

la conductibilité thermique du troisième élément de diffusion de chaleur est de 100 (W/(m-K)) ou plus.

7. Appareil de mesure de flux thermique selon l'une quelconque des revendications précédentes,
un flux thermique est calculé en utilisant les deux ou trois températures parmi la température du premier élément de diffusion de chaleur, la température du deuxième élément de diffusion de chaleur et la température du troisième élément de diffusion de chaleur.

8. Appareil de mesure de flux thermique (4) selon l'une quelconque des revendications précédentes,
dans lequel une section protectrice (47) qui a une ouverture est fournie pour recouvrir la circonférence externe du deuxième élément de diffusion.

9. Appareil de mesure de flux thermique (4) selon la revendication 8,
dans lequel la conductibilité thermique de la section protectrice (47) est inférieure à la conductibilité thermique de la couche de transfert de chaleur.

10. Appareil de mesure de métabolisme (2) comprenant :

l'appareil de mesure de flux thermique (4) selon l'une quelconque des revendications précédentes, et
un appareil informatique (6) qui estime un taux métabolique.

11. Appareil de mesure de température en profondeur comprenant
l'appareil de mesure de flux thermique selon l'une quelconque des revendications 1 à 9.

FIG. 1

FIG. 2

[SEC. A-A]

# FIG. 3

FIG. 4

FIG. 5

FIG. 6

FIG. 7

FIG. 8

$$\left[\begin{array}{c}\text{RELATIONSHIP BETWEEN HEAT FLOW} \\ \text{AND METABOLIC RATE}\end{array}\right]$$

Qall = F(Q)

METABOLIC RATE Qall (kcal / min)

HEAT FLOW Q (kcal / min)

FIG. 9

FIG.10

42, 431, 432, 44, 45    41

6         4

2B

# FIG.11

**EP 2 995 247 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014055963 A
- JP 2007530154 T
- JP 2004532065 T
- JP 2002202205 A
- WO 2013121762 A1